# EUROPEAN PATENT APPLICATION

(11) **EP 4 631 453 A1**
(43) Date of publication of application: **15.10.2025**
(21) Application number: 24211606.9
(22) Date of filing: 08.11.2024
(51) Int. Cl.: A61B 18/14

(54) **SPHINCTEROTOME WITH TWO GUIDE WIRES AND FOUR CAVITIES**

(30) Priority: 09.04.2024 CN 202410421183
(71) Applicant: Anrei Medical (HZ) Co., Ltd., Hangzhou, Zhejiang 310018 (CN)
(72) Inventor: ZHAO, Qiu, Hangzhou City, 310018 (CN); CHEN, Jian, Hangzhou City, 310018 (CN)
(74) Representative: Alatis

(57) **Abstract**

Provided is a sphincterotome with two guide wires and four cavities including a handle assembly, a sheath-tube assembly and a head assembly. The handle assembly includes an electrode electrically connected with a cutting wire. The sheath-tube assembly includes a four-cavity tube internally provided with an injection cavity, a cutting wire cavity and two guide wire cavities which axially run through the four-cavity tube, and an injection inlet communicating with the injection cavity and two guide wire inlets are formed in the four-cavity tube. The head assembly includes a head tube, and an injection outlet communicating with the injection cavity and two guide wire outlets are formed in the head tube. One end, away from the electrode, of the cutting wire passes through the cutting wire cavity to protrude from the head tube, and is fixedly connected to the head tube.

## Description

### TECHNICAL FIELD

The present disclosure relates to the technical field of medical apparatus, in particular to a sphincterotome with two guide wires and four cavities.

### BACKGROUND

With the continuous development of endoscopic technology and the continuous improvement of accessories thereof, ERCP (Endoscopic Retrograde Cholangiopancreatography) has become an important measure for the diagnosis and treatment of hepatobiliary-pancreatic-splenic diseases, such as choledocholithiasis, bile duct injury, bile duct stenosis, biliary complications after liver transplantation. The key to ERCP surgery lies in successful selective intubation of pancreaticobiliary duct and sphincterotomy. For some patients, ERCP surgery fails directly due to intubation failure. Repeated duodenal papilla intubation not only prolongs time of surgery, increasing risk of surgery, but also easily induces acute pancreatitis, increasing the treatment cost and prolonging the hospitalization time, such that great pain is brought to patients, and a certain mortality rate exists. Clinically, in order to improve the success rate of nipple intubation in ERCP surgery, the guide wire is used to assist sphincterotome to perform bile duct intubation, or the pancreatic duct occupation method is usually used for bile duct intubation.

Currently, the sphincterotomy used clinically are mostly sphincterotome equipped with a single guide wire. When the sphincterotome is used for auxiliary intubation, since only one guide wire is used, the entering direction of the guide wire cannot be controlled during auxiliary intubation, resulting that the guide wire enters the pancreatic duct during bile duct intubation, or the guide wire enters the bile duct during pancreatic duct intubation, so that the success rate of selective bile-pancreatic duct intubation is reduced, the time of the surgery is prolonged, duodenal papilla edemas are easily caused, and the incidence of complications such as intraoperative bleeding and postoperative pancreatitis is increased. The sphincterotome is also used in the treatment of right and left hepatic ducts. For example, when the guide wire is located in the right hepatic duct and the pathological position is located in the left hepatic duct, an additional guide wire is needed to be placed, which will repeat the auxiliary intubation of the sphincterotome. Furthermore, there has been one guide wire in the bile duct, the second guide wire cannot be placed into the left hepatic duct efficiently, so that the time of surgery is prolonged, and the incidence of ERCP complications is increased.

In the prior art, Chinese Application Publication NO. CN112587230A discloses a double-guide wire sphincterotome including a main pipeline and a handle, and an opening I is formed in a head end of the main pipe. A first guide wire channel, a second guide wire channel and a third channel are formed in the main pipeline, and the tail of the main pipeline is provided with two extension tubes. An open end of the head of the first guide wire channel is the opening I, and openings of an open end I of the head of the second guide wire channel and an open end II of the head of the third channel are both formed on a side wall of the main pipeline. The guide wire I enters the first guide wire channel through the extension tube and, and the guide wire II enters the second guide wire channel through the extension tube. One end of the electrotome metal wire is fixed on an outer wall of the main pipeline, the electrotome metal wire enters into the third channel from the outside of the main pipeline through the open end II, and then passes through the extension tube and is connected with the handle. In the above-mentioned technical scheme, during papilla intubation or bile duct intubation, the guide wire I enters the pancreatic duct or one of the intrahepatic bile ducts through the head opening, the guide wire II can easily enter the common bile duct or another one of the intrahepatic bile ducts through the side hole by adjusting the direction thereof, but the sphincterotome in such scheme is free of an injection function.

### SUMMARY

Accordingly, the present disclosure aims to provide a sphincterotome with two guide wires and four cavities. According to the sphincterotome with two guide wires and four cavities, simultaneous intubation of the bile duct and the pancreatic duct, or simultaneous guide of guide wires in right and left hepatic ducts, can be achieved more conveniently during intubation, and the sphincterotome has an injection function.

The present disclosure solves the above-mentioned technical problem through following technical means.

A sphincterotome with two guide wires and four cavities includes a handle assembly, a sheath-tube assembly and a head assembly which are connected in sequence. The handle assembly includes a handle body and an electrode arranged on the handle body, and the electrode is connected with a cutting wire. The sheath-tube assembly includes a four-cavity tube internally provided with an injection cavity, a cutting wire cavity and two guide wire cavities which axially run through the four-cavity tube, and an injection inlet communicating with the injection cavity and two guide wire inlets communicating with the two guide wire cavities are formed in the four-cavity tube. The head assembly includes a head tube, and an injection outlet communicating with the injection cavity and two guide wire outlets communicating with the two guide wire cavities are formed in the head tube; and one end, away from the electrode, of the cutting wire passes through the cutting wire cavity to protrude from the head tube, and is fixedly connected to the head tube.

Furthermore, an outer diameter of the head tube is gradually decreased from the sheath-tube assembly to an end, away from the four-cavity tube, of the head tube, and the end of the head tube is hemispherical. In this way, the head assembly is conveniently inserted into the body of a patient, and the end of the head tube is hemispherical so that the head assembly can be smoothly inserted into the body of the patient to avoid from scratching the body of the patient.

Furthermore, the injection inlet is an injection connector arranged on the four-cavity tube and communicating with the injection cavity. The two guide wire inlets comprise a first guide wire connector and a second guide wire connector, the two guide wire cavities comprise a first guide wire cavity and a second guide wire cavity, the first guide wire connector communicates with the first guide wire cavity, and the second guide wire connector communicates with the second guide wire cavity, two guide wire outlets comprise a first guide wire outlet and a second guide wire outlet, the first guide wire outlet connects with the first guide wire cavity, and the second guide wire outlet connects with the second guide wire cavity. During the surgery, two guide wires can be inserted into the two guide wire cavities. In a case that one of the guide wires has been placed in the pancreatic duct, the other of the guide wires can be directly inserted into the bile duct. Alternatively, in a case one of the guide wires has been placed in the right hepatic duct, the other of the guide wires can be directly inserted into the left hepatic duct, so that the success rate of intubation is greatly improved. And the injection inlet is provided as an injection connector, and the guide wire inlet is provided as a guide wire connector, so that the sphincterotome is more convenient to use.

Furthermore, the first guide wire outlet and the injection outlet are arranged on the end, away from the four-cavity tube, of the head tube, the second guide wire outlet is arranged on a side of the head tube, and a distance between the first guide wire outlet and the second guide wire outlet is 3-5 mm. In this way, when one of the guide wires protrudes from the first guide wire outlet, the protruding direction of the guide wire can be consistent with the entire sphincterotome, and the guide wire can be inserted into the pancreatic duct. The other guide wire protrudes from the second guide wire outlet on the side of the head tube, and the protruding direction of the other guide wire can be at a certain slope with the sphincterotome, so that the other guide wire can be inserted into the bile duct other than the pancreatic duct more easily, and the other guide wire can be quickly and accurately inserted.

Furthermore, handle body comprises a core bar, one end of the core bar is provided with a thumb ring, an other end of the core bar is provided with an end cap, and the four-cavity tube is fixedly connected to the end cap. The core bar is provided with a sliding handle, the sliding handle is provided with an electrode holder, and the electrode is mounted on the electrode holder; and the head tube is made of a bendable material. When in use, the index finger and the middle finger is inserted into the sliding handle, and the thumb is inserted into the thumb ring. The sliding handle is slid, and the head tube is bent into a certain arc with the cutting wire to cooperate with the rotation of the duodenoscope, so that the insertion of the guide wires in the first guide wire cavity and the second guide wire cavity is more facilitated.

Furthermore, one end, close to the thumb ring, of the sliding handle is provided with a limiting block slidably arranged on the core bar. In this way, the sliding handle can be limited from sliding excessively on the core bar by means of the limiting block.

Furthermore, an arc-shaped notch is formed at the second guide wire outlet. In this way, a certain inclined angle can be formed conveniently after the guide wire protrudes from the guide wire outlet, and the operation is more convenient.

Furthermore, a distance from the first guide wire connector communicating with the first guide wire outlet to the end of the head tube is larger than a distance from the second guide wire connector to the end of the head tube. In this way, the first guide wire connector is located close to the handle assembly, and the guide wire inserted from the first guide wire connector always protrudes from the end of the head tube, and the other guide wire protrudes from the side of the head tube, so that the doctor is not prone to make errors when performing surgery.

Furthermore, the four-cavity tube is of a circular structure, the four-cavity tube is provided with a first symmetry axis and a second symmetry axis vertical to each other, the first guide wire cavity and the second guide wire cavity are symmetrically arranged on both sides of the first symmetry axis respectively, and the injection cavity and the cutting wire cavity are symmetrically arranged on both sides of the second symmetry axis respectively. In this way, the first guide wire cavity and the second guide wire cavity are located on both sides of the second symmetry axis, so that the two guide wires protrude from the head tube in different angles.

Furthermore, an insulating sleeve sleeves on the cutting wire, and a section, close to the head tube, of the cutting wire is an exposed section. In the technical scheme, only the section, close to the head tube, of the cutting wire is an exposed section, so that the cut larger wound can be avoided, and the patient is safer when the doctor performs surgical cutting.

The present disclosure has the following beneficial effects.

Firstly, the four-cavity tube is internally provided with four cavities including an injection cavity, a cutting wire cavity and two guide wire cavities. When in use, a contrast agent can be directly injected through the injection cavity on the sphincterotome in the present disclosure, the cutting wire in the cutting wire cavity is used for cutting, and two guide wires are inserted through the two guide wire cavities, so that simultaneous intubation of the bile duct and the pancreatic duct, or simultaneous guide of guide wires in right and left hepatic ducts can be achieved more conveniently during intubation, and the sphincterotome is more convenient to use.

Secondly, when in use, the index finger and the middle finger of the medical worker are inserted into the sliding handle, and the thumb of the medical worker is inserted into the thumb ring. The sliding handle is slid toward the thumb ring, and the head tube is bent into a certain arc with the cutting wire to cooperate with the rotation of the duodenoscope. One end of the head tube is inserted into the body of the patient. Since the sphincterotome is provided with two guide wire cavities including the first guide wire cavity and the second guide wire cavity, two guide wires can be inserted through the first guide wire Luer connector and the second guide wire Luer connector. In a case that one of the guide wires has been placed in the pancreatic duct through the first guide wire outlet, the other of the guide wires can be directly obliquely inserted into the bile duct through the second guide wire outlet located on the side of the head tube. Alternatively, in a case that one of the guide wires has been placed in the right hepatic duct through the first guide wire outlet, the other of the guide wires can be directly obliquely inserted into the left hepatic duct through the second guide wire outlet, so that the success rate of intubation is greatly improved, and the incidence of ERCP complications is reduced.

### BRIEF DESCRIPTION OF THE DRAWINGS

FIG. 1 is a structural schematic diagram of a sphincterotome with two guide wires and four cavities in the present disclosure.
FIG. 2 is a structural schematic diagram of a handle assembly in FIG. 1.
FIG. 3 is a structural schematic diagram of a sheath-tube assembly in FIG. 1.
FIG. 4 is a sectional structural schematic diagram of a four-cavity tube in FIG. 3.
FIG. 5 is a structural schematic diagram of a head assembly when a second guide wire outlet is on the left side in FIG. 1.

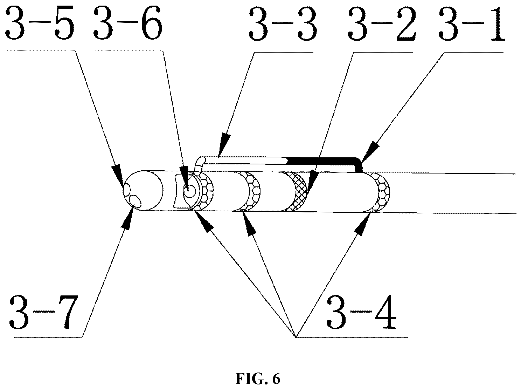

FIG. 6 is a structural schematic diagram of a head assembly when a second guide wire outlet is on the right side in FIG. 1.
FIG. 7 is a top structural schematic diagram of a head assembly in FIG. 6.
FIG. 8 is a schematic diagram of a sphincterotome with two guide wires and four cavities applied at the bile duct and the pancreatic duct in the present disclosure.
FIG. 9 is a schematic diagram of a sphincterotome with two guide wires and four cavities applied at left and right hepatic ducts in the present disclosure.

Reference signs: 1 handle assembly; 2 sheath-tube assembly; 3 head assembly; 4 first guide wire; 5 second guide wire; 1-1 thumb ring; 1-2 core bar; 1-3 limiting block; 1-4 sliding handle; 1-5 electrode holder; 1-6 electrode; 1-7 end cap;
2-1 four-cavity tube; 2-2 first guide wire Luer connector; 2-3 injection Luer connector; 2-4 second guide wire Luer connector; 2-5 heat shrinkage tube; 2-1-1 injection cavity; 2-1-2 first guide wire cavity; 2-1-3 second guide wire cavity; 2-1-4 cutting wire cavity; 2-1-5 first symmetry axis; 2-1-6 second symmetry axis;
3-1 insulating sleeve; 3-2 first color ink scale line; 3-3 cutting wire; 3-4 second color ink scale line; 3-5 first guide wire outlet; 3-6 second guide wire outlet; and 3-7 injection outlet.

### DETAILED DESCRIPTION OF THE EMBODIMENTS

The present disclosure is further described in detail below with reference to the accompanying drawings.

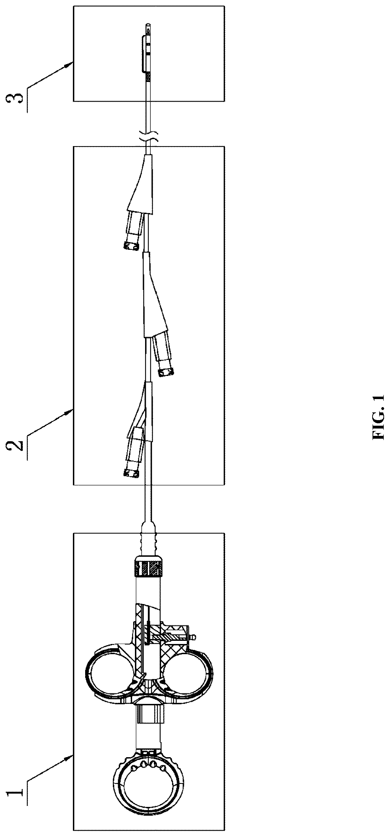

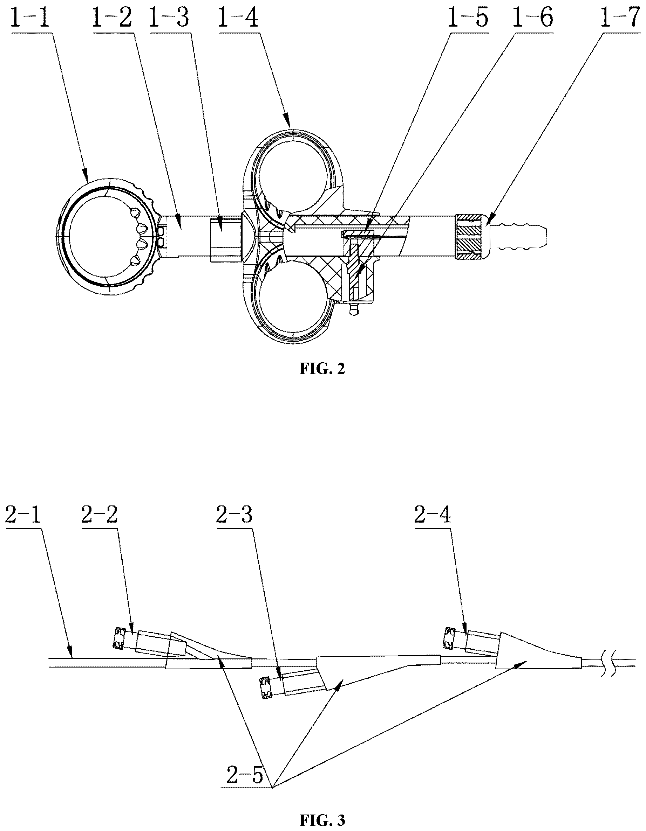

A sphincterotome with two guide wires and four cavities in the embodiment, as shown in FIG. 1, includes a handle assembly 1, a sheath-tube assembly 2 and a head assembly 3 which are connected in sequence. As shown in FIG. 2, the handle assembly 1 includes a handle body and an electrode 1-6, and the electrode 1-6 is electrically connected to a cutting wire 3-3. The handle body includes a core bar 1-2, a left end of the core bar 1-2 is provided with a thumb ring 1-1 connected to the core bar 1-2 via a snap, and a right end of the core bar 1-2 is bonded with an end cap 1-7. A sliding handle 1-4 sleeves on the core bar 1-2 axially and slidably, the sliding handle 1-4 is in threaded connection with an electrode holder 1-5, and the electrode 1-6 is mounted on the electrode holder 1-5 through threads. An end of the sliding handle 1-4 is provided with a limiting block 1-3, and the limiting block 1-3 slidably sleeves on the core bar 1-2.

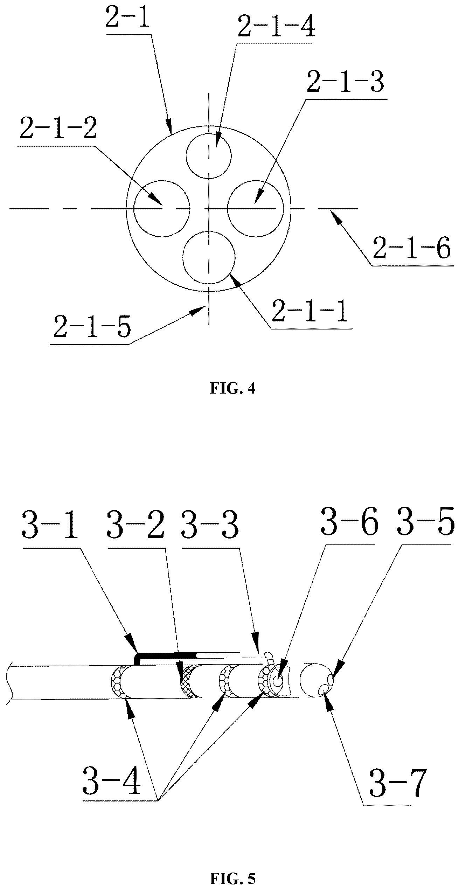

As shown in FIG. 3 and FIG. 4, the sheath-tube assembly 2 includes a four-cavity tube 2-1. The four-cavity tube 2-1 is mounted on the end cap 1-7. The four-cavity tube 2-1 is internally provided with an injection cavity 2-1-1, a cutting wire cavity 2-1-4 and two guide wire cavities which axially run through the four-cavity tube 2-1 and do not communicate with one another. An injection Luer connector 2-3 communicating with the injection cavity 2-1-1 and two guide wire inlets communicating with the two guide wire cavities are formed on the four-cavity tube 2-1. The two guide wire inlets include a first guide wire Luer connector 2-2 and a second guide wire Luer connector 2-4. The joints of the injection Luer connector 2-3, the first guide wire Luer connector 2-2 and the second guide wire Luer connector 2-4 with the four-cavity tube 2-1 are provided with heat shrinkage tubes 2-5. The two guide wire cavities include a first guide wire cavity 2-1-2 and a second guide wire cavity 2-1-3, the first guide wire Luer connector 2-2 is communicated with the first guide wire cavity 2-1-2, and the second guide wire Luer connector 2-4 is communicated with the second guide wire cavity 2-1-3. The two guide wire outlets include a first guide wire outlet 3-5 and a second guide wire outlet 3-6, the first guide wire outlet 3-5 is communicated with the first guide wire cavity 2-1-2, and the second guide wire outlet 3-6 is communicated with the second guide wire cavity 2-1-3. The four-cavity tube 2-1 is of a circular structure. The four-cavity tube 2-1 is provided with a first symmetry axis 2-1-5 and a second symmetry axis 2-1-6 vertical to each other. The first guide wire cavity 2-1-2 and the second guide wire cavity 2-1-3 are symmetrically arranged on both sides of the first symmetry axis 2-1-5 respectively. The injection cavity 2-1-1 and the cutting wire cavity 2-1-4 are symmetrically arranged on both sides of the second symmetry axis 2-1-6 respectively.

The head assembly 3 includes a head tube made of a bendable material, such as a medical rubber material. In the embodiment, the head tube and the four-cavity tube 2-1 are integrally formed. The distance from the first guide wire Luer connector 2-2 communicating with the first guide wire outlet 3-5 to the end of the head tube is larger than the distance from the second guide wire Luer connector 2-4 to the end of the head tube, namely the first guide wire Luer connector 2-2 is closer to the handle assembly 1 than the second guide wire Luer connector 2-4. In the embodiment, the inner diameter φ of a channel of the injection cavity 2-1-1 is 0.7 mm, the inner diameter φ of a channel of the cutting wire cavity 2-1-4 is 0.6 mm, and the inner diameter φ of a channel of each of the first guide wire cavity 2-1-2 and the second guide wire cavity 2-1-3 is 0.75 mm.

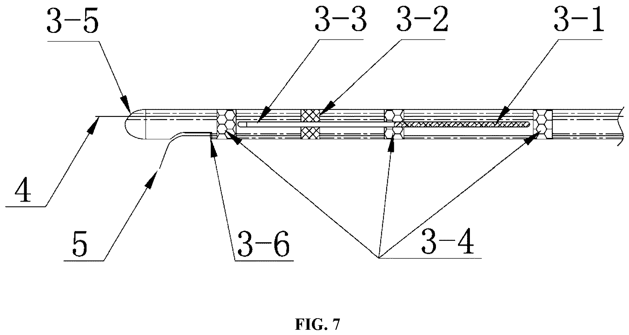

As shown in FIG. 5 to FIGF. 7, the outer diameter of the head tube is gradually decreased from the sheath-tube assembly 2 to a right end of the head tube, and the right end of the head tube is hemispherical. The first guide wire outlet 3-5 and the injection outlet 3-7 are arranged on the hemispherical end of the head tube. The second guide wire outlet 3-6 is arranged on a side of the head tube. As shown in FIG. 5 and FIG. 7, the side of the head tube here can located at a left side or a right side of the cutting wire 3-3. An arc-shaped notch is formed at the second guide wire outlet 3-6 by means of an electric soldering iron or other heating equipment. The distance between the first guide wire outlet 3-5 and the second guide wire outlet 3-6 is 3-5 mm, preferably 4 mm in the embodiment. One end, away from the electrode 1-6, of the cutting wire 3-3 passes through the cutting wire cavity 2-1-4 to protrude from the head tube, and is fixedly connected to the head tube. An insulating sleeve 3-1 sleeves on a section of the cutting wire 3-3, while a section of the cutting wire 3-3 close to the head tube is an exposed section. In order to facilitate a medical worker to know the depth of a portion, inserted into the body, of the head tube, the head tube is also provided with a first color ink scale line 3-2 and a second color ink scale line 3-4.

A using method of the sphincterotome with two guide wires and four cavities in the present disclosure is as follows.

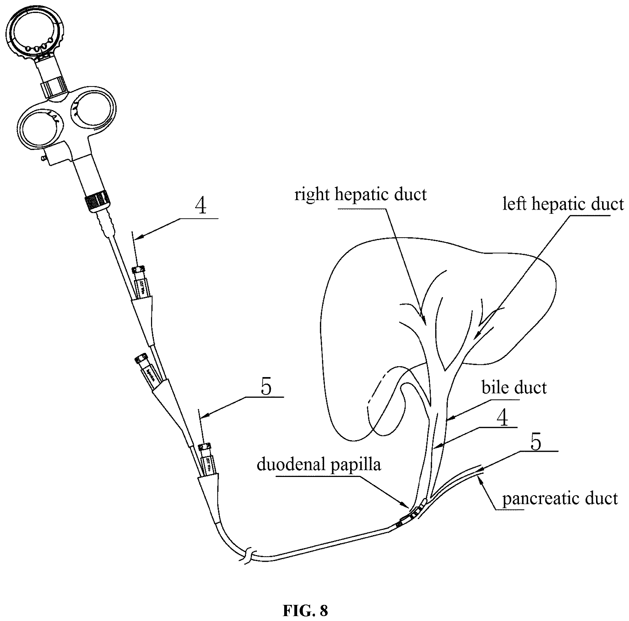

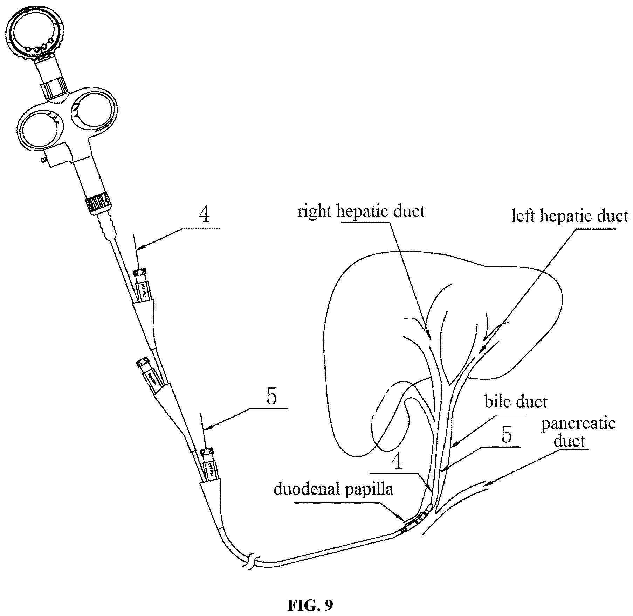

When in use, the index finger and the middle finger of the medical worker are inserted into the sliding handle 1-4, and the thumb of the medical worker is inserted into the thumb ring 1-1. The sliding handle 1-4 is slid toward the thumb ring 1-1, and the head tube is bent into a certain arc with the cutting wire 3-3 to cooperate with the rotation of the duodenoscope. At this time, the limiting block 1-3 can limit the sliding handle 1-4 from excessively sliding on the core bar 1-2. One end of the head tube is inserted into the body of a patient through the duodenoscope, and the medical worker can see the depth of a portion, of inserted into the body of the patient, the head tube through the first color ink scale line 3-2 and the second color ink scale line 3-4. Since the sphincterotome in the embodiment is provided with two guide wire cavities including the first guide wire cavity 2-1-2 and the second guide wire cavity 2-1-3, two guide wires can be inserted through the first guide wire Luer connector 2-2 and the second guide wire Luer connector 2-4. As shown in FIG. 8, in a case that the first guide wire 4 has been placed in the pancreatic duct through the first guide wire outlet 3-5, the second guide wire 5 can be directly obliquely inserted into the bile duct through the second guide wire outlet 3-6 located on the side of the head tube. Alternatively, as shown in FIG. 9, in a case that the first guide wire 4 has been placed in the right hepatic duct through the first guide wire outlet 3-5, the second guide wire 5 can be directly obliquely inserted into the left hepatic duct through the second guide wire outlet 3-6, so that the time of surgery can be greatly reduced, and the incidence of ERCP complications is reduced.

The above-mentioned embodiments are only used for illustrating the technical scheme of the present disclosure but not restricting the scope of protection of the present disclosure; and although the present disclosure is described in detail by reference to a better embodiment, those ordinary skilled in the art should understand that the technical scheme of the present disclosure can be amended or equally substituted but not departing from the purpose and scope of the technical scheme of the present disclosure and should be contained in the scope of claims of the present disclosure. Techniques, shapes and structural parts not described in detail in the present disclosure are all well-known.

## Claims

1. A sphincterotome with two guide wires and four cavities, comprising a handle assembly, a sheath-tube assembly and a head assembly which are connected in sequence, the handle assembly comprising a handle body and an electrode arranged on the handle body, and the electrode being connected with a cutting wire, wherein the sheath-tube assembly comprises a four-cavity tube internally provided with an injection cavity, a cutting wire cavity and two guide wire cavities which axially run through the four-cavity tube, and an injection inlet communicating with the injection cavity and two guide wire inlets communicating with the two guide wire cavities are formed in the four-cavity tube; the head assembly comprises a head tube, and an injection outlet communicating with the injection cavity and two guide wire outlets communicating with the two guide wire cavities are formed in the head tube; and one end, away from the electrode, of the cutting wire passes through the cutting wire cavity to protrude from the head tube, and is fixedly connected to the head tube.

2. The sphincterotome with two guide wires and four cavities according to claim 1, wherein an outer diameter of the head tube is gradually decreased from the sheath-tube assembly to an end, away from the four-cavity tube, of the head tube, and the end of the head tube is hemispherical.

3. The sphincterotome with two guide wires and four cavities according to claim 1, wherein the injection inlet is an injection connector arranged on the four-cavity tube and communicating with the injection cavity; the two guide wire inlets comprise a first guide wire connector and a second guide wire connector, the two guide wire cavities comprise a first guide wire cavity and a second guide wire cavity, the first guide wire connector communicates with the first guide wire cavity, and the second guide wire connector communicates with the second guide wire cavity; and the two guide wire outlets comprise a first guide wire outlet and a second guide wire outlet, the first guide wire outlet communicates with the first guide wire cavity, and the second guide wire outlet communicates with the second guide wire cavity.

4. The sphincterotome with two guide wires and four cavities according to claim 3, wherein the first guide wire outlet and the injection outlet are arranged on the end, away from the four-cavity tube, of the head tube, the second guide wire outlet is arranged on a side of the head tube, and a distance between the first guide wire outlet and the second guide wire outlet is 3-5 mm.

5. The sphincterotome with two guide wires and four cavities according to claim 1, wherein the handle body comprises a core bar, one end of the core bar is provided with a thumb ring, an other end of the core bar is provided with an end cap, and the four-cavity tube is fixedly connected to the end cap; the core bar is provided with a sliding handle, the sliding handle is provided with an electrode holder, and the electrode is mounted on the electrode holder; and the head tube is made of a bendable material.

6. The sphincterotome with two guide wires and four cavities according to claim 5, wherein one end, close to the thumb ring, of the sliding handle is provided with a limiting block slidably arranged on the core bar.

7. The sphincterotome with two guide wires and four cavities according to claim 4, wherein an arc-shaped notch is formed at the second guide wire outlet.

8. The sphincterotome with two guide wires and four cavities according to claim 4, wherein a distance from the first guide wire connector communicating with the first guide wire outlet to the end of the head tube is larger than a distance from the second guide wire connector to the end of the head tube.

9. The sphincterotome with two guide wires and four cavities according to claim 3, wherein the four-cavity tube is of a circular structure, the four-cavity tube is provided with a first symmetry axis and a second symmetry axis vertical to each other, the first guide wire cavity and the second guide wire cavity are symmetrically arranged on both sides of the first symmetry axis respectively, and the injection cavity and the cutting wire cavity are symmetrically arranged on both sides of the second symmetry axis respectively.

10. The sphincterotome with two guide wires and four cavities according to any one of claims 1 to 9, wherein an insulating sleeve sleeves on the cutting wire, and a section, close to the head tube, of the cutting wire is an exposed section.
